# EUROPEAN PATENT APPLICATION

(11) **EP 0 699 669 A1**
(43) Date of publication of application: **06.03.1996**
(21) Application number: 94916403.2
(22) Date of filing: 27.05.1994
(51) Int. Cl.: C07D 249/08, A61K 31/41

(54) **TRIAZOLE DERIVATIVE OR SALT THEREOF, AND FUNGICIDE CONTAINING THE SAME**

(30) Priority: 31.05.1993 JP 129406/93
(71) Applicant: SS PHARMACEUTICAL CO., LTD., Chuo-ku, Tokyo 103 (JP)
(72) Inventor: TOKIZAWA, Minoru, Narita-shi, Chiba 286 (JP); KANAMARU, Yoshihiko, Narita-shi, Chiba 286 (JP); KAWAMOTO, Noriyuki, Narita-shi, Chiba 286 (JP); NAKAZAWA, Hitoshi, Narita-shi, Chiba 286 (JP); NASHIMOTO, Koji, Narita-shi, Chiba 286 (JP); MAEBASHI, Kazuki, Narashino-shi, Chiba 275 (JP); MATSUDA, Hideaki, Abiko-shi, Chiba 270-11 (JP); KURAISHI, Tadayuki, Narashino-shi, Chiba 275 (JP)
(74) Representative: Wächtershäuser, Günter, Prof. Dr.
(86) International application number: JP9400843
(87) International publication number: WO9427976

(57) **Abstract**

The invention relates to a triazole derivative represented by the following general formula (1): 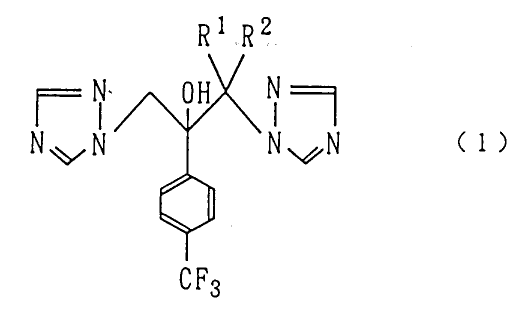
wherein R¹ and R² mean individually a methyl group or denote an ethylene group together, or a salt thereof, and an antifungal agent containing the same.

This compound (1) is high in antifungal activity, and so the antifungal agent contain this derivative as an active ingredient is useful for prevention of and treatment for mycotic infectious diseases incident to mammals including the human.

## Description

### TECHNICAL FIELD

The present invention relates to a triazole derivative, and more particularly to a novel triazole derivative or a salt thereof, which combines an excellent antifungal effect with high safety, and an antifungal agent containing such a derivative or salt as an active ingredient.

### BACKGROUND ART

Mycoses include dermatomycoses typified by various trichophytoses, eczema marginata, psoriases and dermatocandidiases and deep mycoses typified by mycotic meningitis, mycotic infectious diseases of the respiratory organs, fungemia and mycoses of urinary tract.

Of these, the deep mycoses have been difficult to be completely cured by the conventional antibiotics and chemotherapeutants, and so the number of cases thereof has shown a tendency to increase. There has hence been a demand for development of an effective agent for treating these diseases.

As therapeutic agents for such a deep mycosis, there have heretofore been known amphotericin B, fluconazole and the like of the azole type. However, all of them have involved a problem from the viewpoint of safety or antifungal activity. There has thus been a demand for a development of an antifungal agent having high safety and antifungal effect.

### DISCLOSURE OF THE INVENTION

In view of the foregoing circumstances, the present inventors have synthesized a great number of triazole derivatives and salts thereof to investigate their antifungal effects. As a result, it has been found that a triazole derivative represented by the general formula (1), which will be described subsequently, or a salt thereof has an excellent antifungal effect, and at the same time, is high in safety, thus leading to completion of the present invention.

Namely, the present invention provides a triazole derivative represented by the following general formula (1): 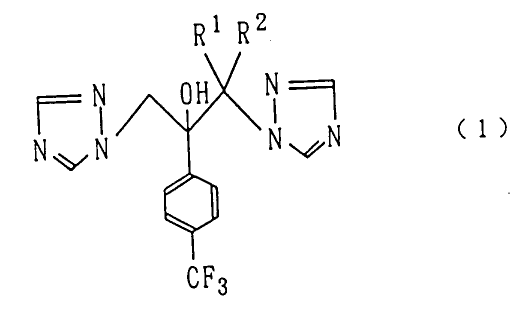
wherein R¹ and R² mean individually a methyl group or denote an ethylene group together, or a salt thereof. The present invention also provides an antifungal agent comprising this triazole derivative (1) or the salt thereof as an active ingredient.

The present invention further provides a medicinal composition comprising the triazole derivative (1) or the salt thereof and a carrier for medicine manufacture.

The present invention still further provides use of this triazole derivative or the salt thereof for a medicine, particularly, a remedy for mycotic infectious diseases.

### BEST MODE FOR CARRYING OUT THE INVENTION

No particular limitation is imposed on the salts of the triazole derivative of the present invention represented by the formula (1) so far as they are pharmaceutically permissible. Examples thereof include the hydrochloride, nitrate, hydrobromide, sulfate, p-toluenesulfonate, methanesulfonate, fumarate, maleate, succinate, lactate and the like.

There are optical isomer based on an asymmetric carbon atom in the compound (1) according to the present invention. However, the present invention includes both racemic modification and optically active substance. The compound (1) according to the present invention also includes hydrates and solvates thereof.

The triazole derivative (1) according to the present invention or a salt thereof may be prepared, for example, in accordance with the following reaction scheme. 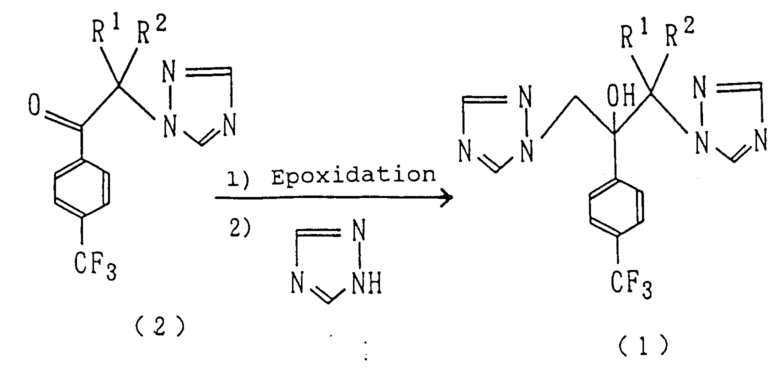
wherein R¹ and R² have the same meaning as defined above.

More specifically, the compound (1) is obtained by reacting 1 mole of the compound (2), 1-2 moles of an epoxidizing agent such as trimethylsulfoxonium iodide and 1-4 moles of triazole in the presence of 2-5 moles of alkali under room temperature to reflux temperature for 1-30 hours.

Examples of the alkali usable in this reaction include sodium hydroxide, potassium hydroxide, barium hydroxide, sodium carbonate and potassium carbonate, with the alkali hydroxides being particularly preferred. Preferable examples of a solvent for the reaction include alcohols such as methanol, ethanol, propanol, isopropanol, butanol, isobutanol, sec-butanol and tert-butanol.

After completion of the reaction, the solvent is distilled off under reduced pressure, and the residue is added with water, extracted with a solvent such as ethyl acetate or chloroform, and dried, followed by distilling off of the solvent. The resultant residue is then purified by column chromatography on silica gel or the like, thereby obtaining the compound (1).

In the general formula (1), the carbon atom, to which a hydroxyl group is bonded, is an asymmetric atom. There are thus two optical isomers based on such an asymmetric atom. These individual optical isomers can be prepared, for example, in accordance with the following reaction scheme. 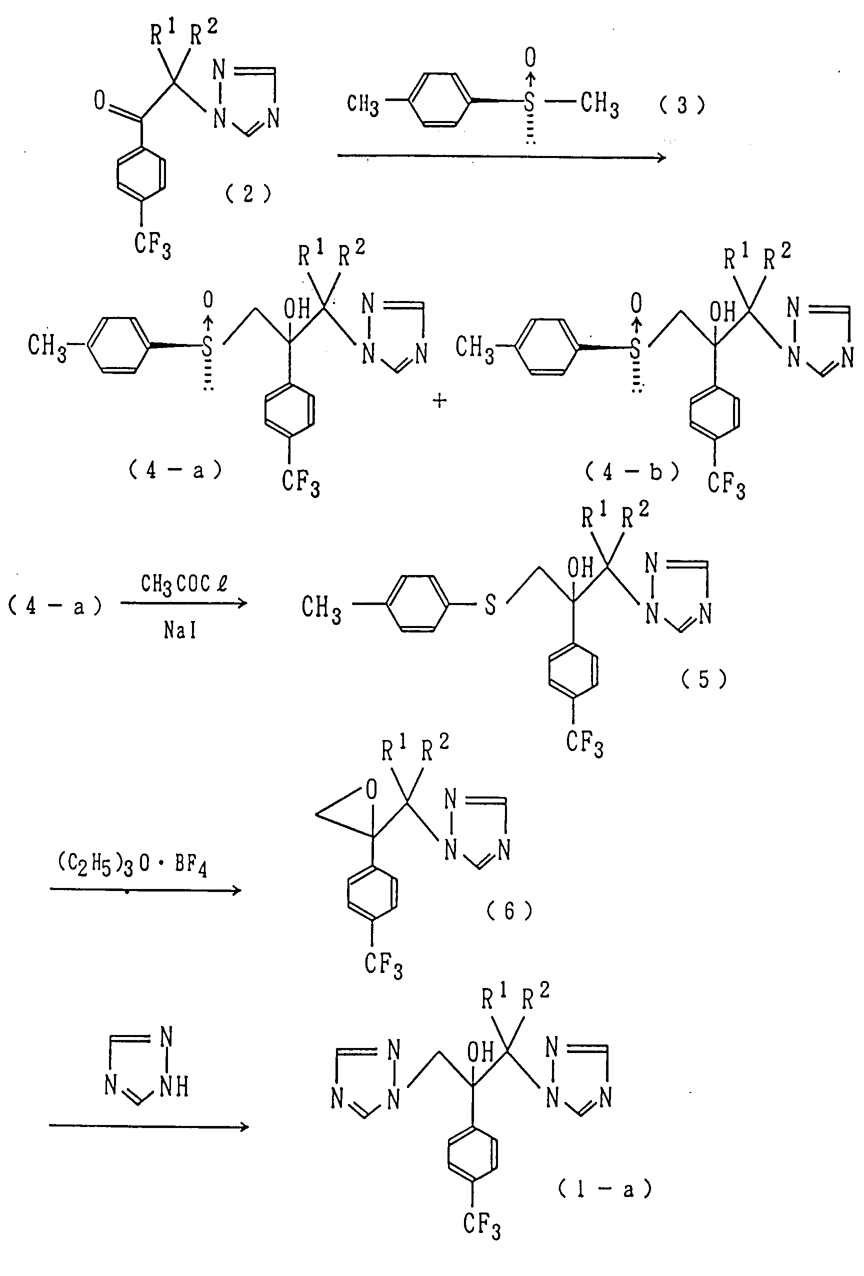
wherein R¹ and R² have the same meaning as defined above, and a compound (4-a) and a compound (4-b) are diastereomers to each other.

More specifically, a compound (2) is reacted with an optically active sulfinyl compound (3) to obtain a mixture of two diastereomers (4-a) and (4-b). One of these diastereomers is separated from this mixture to obtain the compound (4-a). This compound (4-a) is reacted with acetyl chloride and sodium iodide to prepare a compound (5) which is then epoxidized into a compound (6). The compound (6) is then reacted with triazole, whereby an optically active substance (1-a) can be obtained. Following the same procedure, an optically active substance (1-b) can be obtained from the compound (4-b). The optically active substance (1-b) is an antipode to the compound (1-a).

The individual reaction steps will hereinafter be described.

One mole of the compound (2) is reacted with 1-1.2 moles of the optically active sulfinyl compound (3) in the presence of 1-1.4 moles of a base at 0°C to room temperature for 0.5-5 hours, thereby preparing a mixture of the diastereomers (4-a) and (4-b).

Although lithium diisopropylamide, butyl lithium, tert-butoxy potassium, sodium amide, sodium hydride, calcium hydride or the like may be used as the base, lithium diisopropylamide is particularly preferred. As a solvent for the reaction, there may be used ether, tetrahydrofuran, dioxane, hexane or the like.

In order to obtain the intended product from the reaction mixture containing two diastereomers, the mixture is treated with ammonium chloride or the like and extracted with a solvent such as ethyl acetate or chloroform. After the resultant extract is dried, the solvent is distilled off, and the residue is purified by column chromatography on silica gel or the like, whereby the two diastereomers can be separated from each other to separately obtain the diastereomers (4-a) and (4-b).

One mole of the diastereomer (4-a) is dissolved in a solvent such as acetone to react it with 1-5 moles of acetyl chloride and 1-5 moles of sodium iodide at -10°C to room temperature for 0.5-10 hours, thereby preparing the compound (5).

After completion of the reaction, the reaction mixture is added with water and extracted with a solvent such as ether. After the resultant extract is dried, the solvent is distilled off, and the resultant residue is purified by column chromatography on silica gel or the like, thereby obtaining the compound (5).

One mole of the compound is dissolved in a solvent such as chloroform or dichloromethane, to which 2-20 moles of triethyloxonium tetrafluoroborate are then added at 0°C to room temperature for 10-50 hours.
An alkali is added to the reaction mixture to conduct a further reaction at 0°C to room temperature for 0.5-5 hours, thereby preparing the compound (6).

As the alkali, there may be used sodium hydroxide, potassium hydroxide, sodium carbonate, potassium carbonate or the like, with an alkali hydroxide being particularly preferred.

After completion of the reaction, an organic layer is separated and dried, and the solvent is distilled off. The resultant residue is purified by column chromatography on silica gel or the like, thereby obtaining the compound (6).

One mole of the compound (6) is dissolved in a solvent such as dimethylformamide, dimethyl sulfoxide, acetonitrile or tert-butanol, and then reacted with 1-20 moles of triazole in the presence of 1-20 moles of an alkali for 1-50 hours under conditions of room temperature to reflux temperature, thereby preparing the intended, optically active substance (1-a).

As the alkali, there may be used sodium hydroxide, potassium hydroxide, sodium carbonate, potassium carbonate, sodium hydrogencarbonate, potassium hydrogencarbonate or the like.

After completion of the reaction, the reaction mixture is added with water and extracted with a solvent such as ether. After the resultant extract is dried, the solvent is distilled off, and the resultant residue is purified by column chromatography on silica gel or the like and recrystallized from a suitable solvent, thereby obtaining the optically active substance (1-a).

The thus-obtained triazole derivative (1) according to the present invention can be converted into a salt such as the hydrochloride, nitrate, hydrobromide, sulfate, p-toluenesulfonate, methanesulfonate, fumarate, maleate, succinate or lactate, as needed.

Incidentally, the compound (2) as a starting material may be prepared, for example, in accordance with the following reaction scheme (a) or (b).

### Reaction Scheme (a):

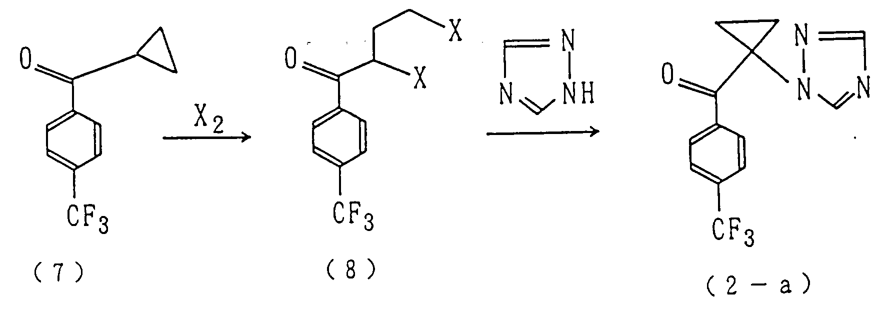

wherein X means a halogen atom.

More specifically, the compound (8) is obtained by the halogenation of cyclopropyl 4-(trifluoromethyl)-phenyl ketone (7), and the compound (2-a) is obtained by the reaction of the compound (8) and triazole.

### Reaction Scheme (b):

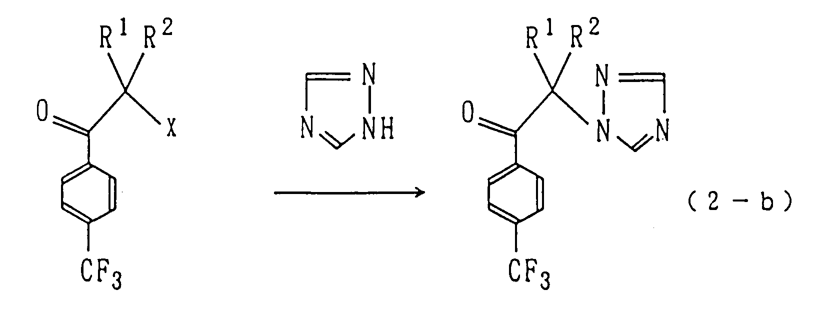

wherein X means a halogen atom, and R¹ and R² denote individually a methyl group.

More specifically, triazole is reacted with 2-halo-4'-(trifluoromethyl)isobutyrophenone (9), thereby obtaining a compound (2-b).

The thus-obtained compound (1) or the salt thereof according to the present invention has an excellent antifungal effect on the pathogenic fungi of dermatomycoses, such as Microsporum, Trichophyton, Achorion, Epidermophyton and Keratinomyces; and the pathogenic fungi of systemic mycoses, such as Candida, Cryptococcus, Aspergilli, Phycomycetes, Sporothrix, Blastomyces and Histoplasma and is high in safety and hence useful as a medicine for treating mycotic infectious diseases.

When the compound (1) or the salt thereof according to the present invention is used as a medicine, it is generally used in the form of a medicinal composition combined with a carrier for medicine manufacture. Examples of the form (preparation form) of such a medicinal composition include tablets, granules, powders, capsules, suspensions, injections, suppositories and external preparations. In order to produce a solid preparation, it is preferable to add an excipient, and optionally a binder, disintegrator, lubricant, colorant, flavor, extender, coating, sugar coating and/or the like to the compound (1) according to the present invention and then form the resultant mixture into tablets, granules, powder, capsules, suppository or the like in accordance with a method known per se in the art. In the case where an injection is prepared, it is only necessary to dissolve, disperse or emulsify the compound (1) according to the present invention in an aqueous carrier such as distilled water for injection in advance or to prepare powder for injection so as to dissolve it in water for injection when used. Administration methods of the injection include intravenous administration, intraarterial administration, intraportal administration, intraperitoneal administration and subcutaneous administration.

When the compound (1) or the salt thereof according to the present invention is administered to the human for the purpose of treatment for a mycotic infectious disease, the dose thereof varies according to the diseased condition, age and weight of the patient to be dosed, dosing route, and the like. However, it is preferably dosed in an amount of generally 1-1,000 mg/day at once or in several installments.

### EXAMPLES

The present invention will hereinafter be described in more detail by the following Examples and Referential Examples. However, it should be borne in mind that this invention is not limited to and by these examples only.

### Referential Example 1:

In 100 ml of acetic acid, were dissolved 43.3 g of cyclopropyl 4-(trifluoromethyl)phenyl ketone. To the resultant solution, 38.8 g of bromine were added dropwise at 40°C, followed by stirring for 4 hours. After completion of the reaction, the reaction mixture was added with cold water and extracted with ether. The resultant organic phase was washed successively with a 10% aqueous solution of potassium carbonate and water. After the thus-washed organic layer was dried over anhydrous magnesium sulfate, the solvent was distilled off, thereby obtaining 68.8 g (yield: 91.4%) of 2,4-dibromo-4'-(trifluoromethyl)butyrophenone as white crystals.
- NMR (CDCl₃) δ ppm:: 2.66(dt,2H), 3.67(t,2H), 5.46(t,1H), 7.79,8.14(d,each 2H).

### Referential Example 2:

To 300 ml of acetonitrile, were added 12.3 g of 1,2,4-triazole and 11.7 g of 85% potassium hydroxide (powder). While cooling with ice water, a solution of 4.3 g of 2,4-dibromo-4'-(trifluoromethyl)butyrophenone in 200 ml of acetonitrile was added dropwise to the mixture, followed by stirring at room temperature for 20 hours. After completion of the reaction, the reaction mixture was added with cold water and extracted with chloroform. The resultant organic layer was washed with water, and dried over anhydrous magnesium sulfate. Then the solvent was distilled off, and the residue was purified by column chromatography on silica gel with chloroform/n-hexane (1:1), thereby obtaining 16.9 g (yield: 74.6%) of 1-(1H-1,2,4-triazol-1-yl)cyclopropyl 4-(trifluoromethyl)phenyl ketone as white crystals. Melting point: 68-70°C.
- NMR (CDCl₃) δ ppm:: 1.7-1.9,2.0-2.2(m,each 2H), 7.56(s,4H), 7.92,8.11(d,each 1H).

### Example 1:

To 30 ml of tert-butanol, were added 0.56 g of 1-(1H-1,2,4-triazol-1-yl)cyclopropyl 4-(trifluoromethyl) phenyl ketone, 0.58 g of trimethylsulfoxonium iodide, 0.32 g of 1,2,4-triazole and 0.39 g of 85% potassium hydroxide (powder), and the mixture was stirred at 80°C for 6 hours. After completion of the reaction, the solvent was distilled off, and the residue was added with cold water and extracted with chloroform. The resultant organic layer was washed with water and dried over anhydrous magnesium sulfate. Thereafter, the solvent was distilled off, and the residue was purified by column chromatography on silica gel with chloroform, thereby obtaining 0.41 g (yield: 56.9%) of 2-(1H-1,2,4-triazol-1-yl)-1-[1-(1H-1,2, 4-triazol-1-yl)cyclopropyl]-1-[4-(trifluoromethyl)phenyl]-ethan-1-ol (Compound No. 1-1) as white crystals. Melting point: 162-164°C.
IR (KBr) ν cm⁻¹: 1330, 1120.
- NMR (CDCl₃) δ ppm:: 0.8-1.7(m,4H), 4.85,5.03(s,each 1H), 7.07,7.49(s,each 2H), 7.36,7.93,8.01,8.53(s,each 1H).

### Referential Example 3:

In 40 ml of acetonitrile, were dissolved 5.00 g of 2-bromo-4'-(trifluoromethyl)isobutyrophenone, to which 5.00 g of 1,2,4-triazole and 2.30 g of potassium hydroxide were added. The resultant mixture was refluxed for 1.5 hours. After completion of the reaction, the solvent was distilled off, and the residue was added with cold water and extracted with ethyl acetate. The resultant organic layer was washed with water. After the thus-washed organic layer was dried over anhydrous magnesium sulfate, the solvent was distilled off, and the residue was purified by column chromatography on silica gel with chloroform, thereby obtaining 3.90 g (yield: 74.6%) of 2-(1H-1,2,4-triazol-1-yl)-4'-(trifluoromethyl)isobutyropheno ne as white crystals. Melting point: 85-87°C.
- NMR (CDCl₃) δ ppm:: 1.95(s,6H), 7.42,7.55(d,each 2H), 7.98,8.30(s,each 1H).

### Example 2:

To 30 ml of isopropanol, were added 1.13 g of 2-(1H-1, 2,4-triazol-1-yl)-4'-(trifluoromethyl)isobutyrophenone, 0.90 g of trimethylsulfoxonium iodide, 0.90 g of 1,2,4-triazole and 0.50 g of 85% potassium hydroxide (powder), and the resultant mixture was heated under reflux for 2.5 hours. After completion of the reaction, the solvent was distilled off, and the residue was added with cold water and extracted with ethyl acetate. The resultant organic layer was washed with water and dried over anhydrous magnesium sulfate. Thereafter, the solvent was distilled off, and the residue was purified by column chromatography on silica gel with chloroform/methanol (100:3), thereby obtaining 0.40 g (yield: 27.3%) of 3-methyl-1,3-bis(1H-1,2,4-triazol-1-yl)-2-[4-(trifluoromethyl)phenyl]butan-2-ol (Compound No. 1-4) as white crystals. Melting point: 162-163°C.
- NMR (CDCl₃) δ ppm:: 1.63,1.81(s,each 3H), 4.53,5.35(d,each 1H), 6.8-7.2(broad,2H), 7.41(d,2H), 7.22,7.87,7.89,8.08(s,each 1H).

### Example 3:

A solution of 6.78 g of S(-)methyl-p-tolyl sulfoxide in 40 ml of tetrahydrofuran was added dropwise to 34.8 ml of a 1.5 M cyclohexane solution of lithium diisopropylamide at room temperature, and the mixture was stirred for 2 hours. A solution of 11.24 g of 1-(1H-1,2,4-triazol-1-yl)cyclopropyl 4-(trifluoromethyl)phenyl ketone in 40 ml of tetrahydrofuran was then added dropwise, followed by further stirring for 2 hours. After completion of the reaction, the reaction mixture was added with a saturated aqueous solution of ammonium chloride and extracted with ethyl acetate. The resultant organic layer was washed with water and dried over anhydrous magnesium sulfate. Thereafter, the solvent was distilled off, and the residue was purified by column chromatography on silica gel with toluene/n-hexane (100:3), thereby obtaining two diastereomers (Compound No. 4-1) and (Compound No. 4-2) of α-(4-methylbenzosulfinyl)methyl-α-[1-(1H-1,2,4-triazol-1-yl)]cyclopropyl-4-(trifluoromethyl)benzyl alcohol as white crystals. Compound (4-1): amount produced: 5.77 g (yield: 33.2%), melting point: 184-185°C. [α]_{D}²⁰ = -132.6° (acetone, c = 0.5). IR (KBr) ν cm⁻¹: 1331, 1120.
- NMR (CDCl₃) δ ppm:: 0.9-1.1,1.3-1.4,1.6-1.7,2.0-2.2(m,each 1H), 2.40(s.3H), 3.15,3.90(d,each 1H), 6.29(s,1H), 6.8-7.6(m,8H), 7.38,7.99(s,each 1H).
- Compound (4-2):: amount produced: 3.98 g (yield: 22.9%), melting point: 72-74°C.
[α]_{D}²⁰ = +0.3° (acetone, c = 0.5). IR (KBr) ν cm⁻¹: 1325, 1128.
- NMR (CDCl₃) δ ppm:: 0.9-1.1(m,2H), 1.5-1.6,1.6-1.8(m,each 1H), 2.45(s.3H), 3.33,3.93(d,each 1H), 5,94(s,1H), 7.2-7.7(m,8H), 7.42,7.86(s,each 1H).

### Example 4:

In 30 ml of acetone, were dissolved 3.58 g of (-)-α-(4-methylbenzosulfinyl)methyl-α-[1-(1H-1,2,4-triazol-1-yl)]cyclopropyl-4-(trifluoromethyl)benzyl alcohol (4-1), to which 1.94 g of acetyl chloride were added dropwise while cooling with ice water, followed by addition of 3.70 g of sodium iodide. The mixture was stirred at 0-10°C for 1 hour. After completion of the reaction, the reaction mixture was added with cold water and extracted with ether. The resultant organic layer was washed successively with a saturated aqueous solution of sodium hydrogencarbonate, a saturated aqueous solution of sodium thiosulfate and water and dried over anhydrous magnesium sulfate. The solvent was then distilled off, and the residue was crystallized from isopropyl ether/n-hexane (1:1), thereby obtaining 2.71 g (yield: 78.6%) of (-)-α-[ (4-methylphenyl)thio]methyl-α-[1-(1H-1,2,4-triazol-1-yl)]-cyclopropyl-4-(trifluoromethyl)benzyl alcohol as white crystals. Melting point: 113-115°C.
[α]_{D}²⁰ = -59.0° (acetone, c = 0.5). IR (KBr) ν cm⁻¹: 1329, 1126.
- NMR (CDCl₃) δ ppm:: 0.9-1.8(m,4H), 2.26(s.3H), 3.38,4.04(d,each 1H), 3.73(s,1H), 6.8-7.5(m,8H), 7.51,7.87(s,each 1H).

### Example 5:

In 20 ml of acetone, were dissolved 1.30 g of (+)-α-(4-methylbenzosulfinyl)methyl-a-[1-(1H-1,2,4-triazol -1-yl)]cyclopropyl-4-(trifluoromethyl)benzyl alcohol (4-2), to which 0.70 g of acetyl chloride was added dropwise while cooling with ice water, followed by addition of 1.54 g of sodium iodide. The mixture was stirred at 0-10°C for 6 hours. After completion of the reaction, the reaction mixture was added with cold water and extracted with ether. The resultant organic layer was washed successively with a saturated aqueous solution of sodium hydrogencarbonate, a saturated aqueous solution of sodium thiosulfate and water and dried over anhydrous magnesium sulfate. The solvent was then distilled off, and the residue was crystallized from isopropyl ether/n-hexane (1:1), thereby obtaining 1.00 g (yield: 79.9%) of (+)-α-[(4-methylphenyl)thio]methyl-α-[1-(1H-1,2,4-triazol-1-yl)]cyclopropyl-4-(trifluoromethyl)benzyl alcohol as white crystals. Melting point: 113-115°C.
[α]_{D}²⁰ = +59.0° (acetone, c = 0.5).
IR (KBr) ν cm⁻¹: 1329, 1126.
- NMR (CDCl₃) δ ppm:: 1.0-1.9(m,4H), 2.26(s.3H), 3.36,4.08(d,each 1H), 3.78(s,1H), 6.8-7.5(m,8H), 7.56,7.90(s,each 1H).

### Example 6:

In 5 ml of dichloromethane, were dissolved 419 mg of (-)-α-[(4-methylphenyl)thio]methyl-α-[1-(1H-1,2,4-triazol-1-yl)]cyclopropyl-4-(trifluoromethyl)benzyl alcohol, to which 13 ml of a 1 M dichloromethane solution of triethyloxonium tetrafluoroborate were added at room temperature. The mixture was stirred for 48 hours. While cooling with ice water, 10 ml of a 20% aqueous solution of sodium hydroxide were then added, followed by stirring for 1 hour. After completion of the reaction, the resultant organic phase was separated and washed with water. After the thus-washed organic layer was dried over anhydrous magnesium sulfate, the solvent was distilled off, and the residue was purified by column chromatography on silica gel with n-hexane/ethyl acetate (5:1), thereby obtaining 206 mg (yield: 69.8%) of (+)-2-[1-(1H-1,2,4-triazol-1-yl)]cyclopropyl-2-[4-(trifluoro methyl)phenyl]oxirane as white crystals. Melting point: 61-62°C.
[α]_{D}²⁰ = +5.2° (acetone, c = 0.25).
IR (KBr) ν cm⁻¹: 1325, 1124.
- NMR (CDCl₃) δ ppm:: 1.1-1.5(m,4H), 2.94,3.20(d,each 1H), 7.25,7.54(d,each 2H), 7.87,7.92(s,each 1H),

### Example 7:

In 5 ml of dichloromethane, were dissolved 419 mg of (+)-α-[(4-methylphenyl)thio]methyl-α-[1-(1H-1,2,4-triazol-1-yl)]cyclopropyl-4-(trifluoromethyl)benzyl alcohol, to which 15 ml of a 1 M dichloromethane solution of triethyloxonium tetrafluoroborate were added at room temperature. The mixture was stirred for 18 hours. While cooling with ice water, 10 ml of a 20% aqueous solution of sodium hydroxide were then added, followed by stirring for 1 hour. After completion of the reaction, the resultant organic layer was separated and washed with water. After the thus-washed organic layer was dried over anhydrous magnesium sulfate, the solvent was distilled off, and the residue was purified by column chromatography on silica gel with n-hexane/ethyl acetate (5:1), thereby obtaining 170 mg (yield: 57.6%) of (-)-2-[1-(1H-1,2,4-triazol-1-yl)]cyclopropyl-2-[4-(trifluoro methyl)phenyl]oxirane as a colorless oil. Melting point: 61-62°C.
[α]_{D}²⁰ = -5.2° (acetone, c = 0.25). IR (neat) ν cm⁻¹: 1327, 1127.
- NMR (CDCl₃) δ ppm:: 1.1-1.5(m,4H), 2.94,3.15(d,each 1H), 7.26,7.58(d,each 2H), 7.88,7.92(s,each 1H),

### Example 8:

In 4 ml of dimethylformamide, were dissolved 173 mg of (+)-2-[1-(1H-1,2,4-triazol-1-yl)]cyclopropyl-2-[4-(trifluoro methyl)phenyl]oxirane and 404 mg of 1,2,4-triazole, to which 178 mg of potassium carbonate were added at room temperature. The mixture was stirred for 48 hours. After completion of the reaction, the reaction mixture was added with cold water and extracted with ether. The resultant organic layer was washed with water and dried over anhydrous magnesium sulfate. Thereafter, the solvent was distilled off, and the residue was purified by column chromatography on silica gel with chloroform and further recrystallized from a 40% aqueous solution of ethanol, thereby obtaining 72 mg (yield: 37.1%) of (+)-2-(1H-1,2,4-triazol-1-yl)-1-[1-(1H-1,2,4-triazol-1-yl)cyclopropyl]-1-[4-(trifluoromethyl)phenyl]ethan-1-ol (Compound No. 1-2) as white crystals. Melting point: 153-155°C.
[α]_{D}²⁰ = +7.2° (acetone, c = 0.25).
IR (KBr) ν cm⁻¹: 1327, 1120.
- NMR (CDCl₃) δ ppm:: 0.9-1.0(m,3H), 1.5-1.6(m,1H), 4.85,5.07(d,each 1H), 7.07,7.51(d,each 2H), 7.37,7.94,8.02,8.58(s,each 1H).

### Example 9:

In 4 ml of dimethylformamide, were dissolved 320 mg of (-)-2-[1-(1H-1,2,4-triazol-1-yl)]cyclopropyl-2-[4-(trifluoro methyl)phenyl]oxirane and 748 mg of 1,2,4-triazole, to which 328 mg of potassium carbonate were added at room temperature. The mixture was stirred for 49 hours. After completion of the reaction, the reaction mixture was added with cold water and extracted with ether. The resultant organic layer was washed with water and dried over anhydrous magnesium sulfate. Thereafter, the solvent was distilled off, and the residue was purified by column chromatography on silica gel with chloroform and further recrystallized from a 40% aqueous solution of ethanol, thereby obtaining 209 mg (yield: 52.9%) of (-)-2-(1H-1,2,4-triazol-1-yl)-1-[1-(1H-1,2,4-triazol-1-yl)cyclopropyl]-1-[4-(trifluoromethyl)phenyl]-ethan-1-ol (Compound No. 1-3) as white crystals. Melting point: 153-155°C.
[α]_{D}²⁰ = -7.3° (acetone, c = 0.25).
IR (KBr) ν cm⁻¹: 1327, 1120.
- NMR (CDCl₃) δ ppm:: 0.9-1.0(m,3H), 1.5-1.6(m,1H), 4.84,5.12(d,each 1H), 7.10,7.30(d,each 2H), 7.38,7.96,8.04,8.58(s,each 1H).

### Example 10:

The germination-inhibiting effects in vitro of the typical triazole compounds according to the present invention on a Candida albicans ATCC44895 strain were determined.

More specifically, an Eagle's minimum essential medium added with a 10% fetal bovine serum was infected with the Candida albicans ATCC44895 strain so as to give a concentration of 2 x 10⁵ cfu/ml, thereby incubating it at 37°C for 20 hours in a CO₂-incubator. Thereafter, the germination-inhibiting effect was tested as to the cases where no agent was added and where each of the test agents was added with the agent diluted from the maximum concentration of 1,000 ng/ml in a common ratio of 2.

Compound No. (1-1) and Compound No. (1-3) showed a germination-inhibiting effect in concentrations of at least 125 ng/ml and at least 62.5 ng/ml, respectively. The control agent (fluconazole) showed a germination-inhibiting effect in a concentration of at least 250 ng/ml. As described above, the compounds according to the present invention exhibited stronger germination-inhibiting effects on Candida albicans than the control agent.

### Example 11: (Tablet preparation)

| | |
|---|---|
| Compound (1-3) | 50 mg |
| Crystalline cellulose | 50 mg |
| Lactose | 50 mg |
| Hydroxypropylcellulose | 18 mg |
| Magnesium stearate | 2 mg |
| Total | 170 mg |

A tablet preparation composed of the above composition was formulated in accordance with a method known per se in the art. This tablet preparation may be provided as a sugar-coated tablet preparation or a film-coated tablet preparation as needed.

### Example 12: (Capsulated preparation)

| | |
|---|---|
| Compound (1-3) | 50 mg |
| Precipitated silicic anhydride | 25 mg |
| Lactose | 100 mg |
| Starch | 50 mg |
| Talc | 25 mg |
| Total | 250 mg |

The above components were placed into No. 1 capsules to obtain a capsulated preparation.

### Example 13: (Granulated preparation)

| | |
|---|---|
| Compound (1-1) | 50 mg |
| Lactose | 600 mg |
| Corn starch | 200 mg |
| Sodium carboxymethylcellulose | 20 mg |
| Hydroxypropylcellulose | 130 mg |
| Total | 1000 mg |

A granulated preparation composed of the above composition was formulated in accordance with a method known per se in the art.

### Example 14: (Powdered preparation)

| | |
|---|---|
| Compound (1-1) | 50 mg |
| Precipitated silicic anhydride | 20 mg |
| Precipitated calcium carbonate | 10 mg |
| Lactose | 250 mg |
| Starch | 70 mg |
| Total | 400 mg |

A powdered preparation composed of the above composition was formulated in accordance with a method known per se in the art.

### Example 15: (Injection preparation)

| | |
|---|---|
| Compound (1-3) | 5 mg |
| Hardened castor oil | 85 mg |
| Propylene glycol | 60 mg |
| Glucose | 50 mg |
| Distilled water for injection | To 1 ml in total |

An injection preparation composed of the above composition was formulated in accordance with a method known per se in the art.

### Example 16: (Injection preparation)

| | |
|---|---|
| Compound (1-3) | 5 mg |
| Polyoxyethylene hardened castor oil | 40 mg |
| Propylene glycol | 60 mg |
| Distilled water for injection | To 1 ml in total |

An injection preparation composed of the above composition was formulated in accordance with a method known per se in the art.

### INDUSTRIAL APPLICABILITY

The triazole derivative (1) according to the present invention is high in antifungal activity and easy to be absorbed and hence high in availability in vivo. Therefore, an antifungal agent containing this derivative as an active ingredient is useful for prevention of and treatment for mycotic infectious diseases incident to mammals including the human.

## Claims

1. A triazole derivative represented by the following general formula (1): 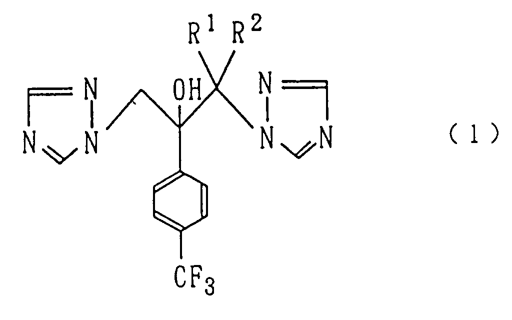 wherein R¹ and R² mean individually a methyl group or denote an ethylene group together, or a salt thereof.

2. An antifungal agent comprising, as an active ingredient, the triazole derivative or the salt thereof according to Claim 1.

3. A medicinal composition comprising the triazole derivative or the salt thereof according to Claim 1 and a carrier for medicine manufacture.

4. Use of the triazole derivative or the salt thereof according to Claim 1 for a medicine

5. Use of the triazole derivative or the salt thereof according to Claim 1 for a remedy for mycotic infectious diseases.
